# EUROPÄISCHE PATENTSCHRIFT

(11) **EP 3 892 228 B1**
(45) Veröffentlichungstag und Bekanntmachung des Hinweises auf die Patenterteilung: **08.05.2024**
(21) Anmeldenummer: 21167408.0
(22) Anmeldetag: 08.04.2021
(51) Int. Cl.: A61B 90/90, A61B 90/92

(54) **MEDIZINISCHES INSTRUMENT ODER TEIL EINES MEDIZINISCHEN INSTRUMENTS**
MEDICAL INSTRUMENT OR PART OF A MEDICAL INSTRUMENT
INSTRUMENT MÉDICAL OU PARTIE D'INSTRUMENT MÉDICAL

(30) Priorität: 09.04.2020 DE 102020204580
(43) Veröffentlichungstag der Anmeldung: 13.10.2021
(73) Patentinhaber: Aesculap AG, 78532 Tuttlingen (DE)
(72) Erfinder: Mattes, Ursula, 78603 Renquishausen (DE)
(74) Vertreter: Patentanwälte Ruff, Wilhelm, Beier, Dauster & Partner mbB

(56) Entgegenhaltungen:
- EP-B1- 2 012 682
- CN-A- 108 836 516
- DE-U1-202019 101 070
- DE-U1-212008 000 085

## Beschreibung

### ANWENDUNGSGEBIET UND STAND DER TECHNIK

Die Erfindung betrifft ein medizinisches, insbesondere chirurgisches, Instrument oder ein Teil eines medizinischen, insbesondere chirurgischen, Instruments.

Die Unterscheidung von insbesondere ähnlich aussehenden medizinischen Instrumenten bereitet regelmäßig Schwierigkeiten. Von Relevanz ist dies vor allem bei chirurgischen Instrumenten. Beispielsweise sind Sägeblöcke, welche bei einer Kniegelenksoperation zum Einsatz kommen, aufgrund ihrer minimalen Größenunterschiede nur schwer voneinander zu unterscheiden.

Ferner bereitet die Unterscheidbarkeit von medizinischen Instrumenten regelmäßig Probleme bei zentralen Sterilgutversorgungsabteilungen (ZSVA), wo medizinische Instrumente jeweiligen Sterilisationssieben zugeordnet werden müssen.

Gegenstand von DE20 2019 101070 U1 ist ein Positionsführungssystem für ein chirurgisches Instrument. Das Positionsführungssystem weist eine als ringförmige Hohleinheit gestaltete Anzeigeeinheit mit einem Markerfluid auf. Die Anzeigeeinheit des Positionsführungssystems ist mit einer Hub-Einheit des chirurgischen Instruments verbindbar. Bei dem chirurgischen Instrument kann es sich beispielsweise um eine Nadel handeln.

### AUFGABE UND LÖSUNG

Der vorliegenden Erfindung liegt daher die Aufgabe zugrunde, ein medizinisches, insbesondere chirurgisches, Instrument oder ein Teil eines solchen Instruments sowie ein Kit oder Set bereitzustellen, welches eine bessere Unterscheidbarkeit/Erkennbarkeit von medizinischen Instrumenten oder medizinischen Instrumententeilen im Vergleich zu gattungsgemäßen Instrumenten oder Instrumententeilen ermöglicht, insbesondere bei konstruktiv gleich oder ähnlich ausgebildeten medizinischen, insbesondere chirurgischen, Instrumenten oder Instrumententeilen.

Diese Aufgabe wird gelöst durch ein medizinisches Instrument gemäß unabhängigem Anspruch 1 sowie durch ein Kit oder Set gemäß Anspruch 12.

Bevorzugte Ausgestaltungen sind in den abhängigen Ansprüchen definiert. Der Wortlaut sämtlicher Ansprüche wird hiermit durch ausdrückliche Bezugnahme zum Inhalt der vorliegenden Beschreibung gemacht.

Die Erfindung betrifft ein medizinisches Instrument oder ein Teil eines medizinischen Instruments. Bei dem medizinischen Instrument handelt es sich vorzugsweise um ein chirurgisches Instrument, d.h. um ein Instrument, welches in der Chirurgie und/oder in der Orthopädie, insbesondere chirurgischen Orthopädie, zum Einsatz kommt bzw. Verwendung findet. Entsprechend handelt es sich bei dem Teil eines medizinischen Instruments bevorzugt um ein Teil eines chirurgischen Instruments.

Das medizinische Instrument oder das Teil eines medizinischen Instruments zeichnet sich besonders dadurch aus, dass es einen geschlossenen, insbesondere hermetisch, d.h. luft- und/oder flüssigkeitsdicht, geschlossenen, Hohlkörper aufweist. Der Hohlkörper ist zudem wenigstens abschnittsweise, insbesondere nur abschnittsweise oder vollständig, blick- oder lichtdurchlässig gestaltet. Ferner enthält der Hohlkörper ein Kennzeichnungselement, wobei das Kennzeichnungselement durch den wenigstens abschnittsweise blick- oder lichtdurchlässig gestalteten Hohlkörper hindurch einsehbar oder erkennbar, insbesondere ablesbar, ist. Mit anderen Worten ist das Kennzeichnungselement aufgrund des wenigstens abschnittsweise blick- oder lichtdurchlässig gestalteten Hohlkörpers von außen einsehbar oder erkennbar, insbesondere ablesbar.

Ferner kann eine Außenoberfläche, vorzugsweise eine beim bestimmungsgemäßen Gebrauch des medizinischen Instruments oder des Teils eines medizinischen Instruments sichtbare Außenoberfläche, des Hohlkörpers Markierungen, insbesondere in Form einer Skalierung oder Ähnlichem, aufweisen. Dadurch kann die Erkennbarkeit und/oder Unterscheidbarkeit des medizinischen Instruments oder des Teils eines medizinischen Instruments gegenüber anderen Instrumenten oder anderen Instrumententeilen zusätzlich verbessert werden.

Unter dem Ausdruck "blickdurchlässig" oder "lichtdurchlässig" soll im Sinne der vorliegenden Erfindung die Durchlässigkeit in Bezug auf elektromagnetische Wellen des Lichts, d.h. in Bezug auf elektromagnetische Wellen, die für das menschliche Auge sichtbar sind, vorzugsweise in Bezug auf elektromagnetische Wellen mit einer Wellenlänge von 380 nm bis 780 nm, vorzugsweise 400 nm bis 700 nm, verstanden werden.

Die Erfindung zeichnet sich insbesondere durch nachfolgende Vorteile aus:
- Durch das erfindungsgemäß vorgesehene einseh- oder erkennbare Kennzeichnungselement vereinfacht sich die Handhabung, insbesondere die Erkennbarkeit/Unterscheidbarkeit, des erfindungsgemäßen Instruments oder Instrumententeils für einen Anwender, insbesondere einen Arzt, für OP-Personal sowie für Mitarbeiter in zentralen Sterilgutversorgungsabteilungen, signifikant. Dadurch lassen sich beispielsweise OP-Zeiten sowie Durchlaufzeiten in zentralen Sterilgutversorgungsabteilungen deutlich verkürzen.
- Ein weiterer Vorteil besteht darin, dass prinzipiell eine breite Palette an Kennzeichnungselementen in Betracht kommt. Somit sind über eine gezielte Auswahl eines oder mehrerer Kennzeichnungselemente kundenspezifische Lösungen realisierbar, die sich beispielsweise durch Sterilisationsbeständigkeit und/oder Säurebeständigkeit und/oder Biokompatibilität auszeichnen und/oder eine einfachere und/oder schnellere Zuordnung ermöglichen.
- Ein weiterer Vorteil besteht darin, dass der Hohlkörper in Bezug auf seine Abmessungen (Größe) und/oder Form keinerlei Einschränkungen unterliegt und mithin gezielt an ein medizinisches Instrument oder ein Teil eines medizinischen Instruments angepasst werden kann.
- Ein weiterer Vorteil besteht darin, dass der Hohlkörper mit dem darin enthaltenen Kennzeichnungselement als (weiteres) Bauteil einfach in/an ein entsprechend zu markierendes medizinische Instrument oder in/an ein entsprechend zu markierendes Teil eines medizinischen Instruments eingebaut/montiert werden kann, beispielsweise durch Ein- oder Verpressen, Kleben, Verstemmen, Clipsen oder Klinchen.
- Ferner ist von Vorteil, dass der Hohlkörper mit dem darin enthaltenen Kennzeichnungselement spaltfrei in/an ein entsprechend zu markierendes medizinisches Instrument oder in/an ein entsprechend zu markierendes Teil eines medizinischen Instruments eingebaut/montiert werden kann. Dadurch kann mit besonderem Vorteil eine Reinigung und/oder Aufbereitung des medizinischen Instruments oder des Teils eines medizinischen Instruments verbessert werden.
- Dadurch, dass das Kennzeichnungselement in einem geschlossenen Hohlkörper enthalten und mithin von exogenen Einflüssen abgeschirmt oder im Wesentlichen/weitgehend abgeschirmt ist, kann zum Beispiel eine Rissbildung und/oder Versprödung des Kennzeichnungselements vollständig oder im Wesentlichen/weitgehend vermieden werden. Dadurch besteht prinzipiell die Möglichkeit, das medizinische Instrument oder das Teil eines medizinischen Instruments, insbesondere nach einer entsprechenden Reinigung und/oder Aufbereitung, mehrmals zu verwenden.
- Handelt es sich bei dem Kennzeichnungselement beispielsweise um einen Feststoff, worauf im Folgenden noch näher eingegangen werden wird, besteht ein weiterer Vorteil darin, dass der Feststoff mit Bildern, Logos, Zeichnungen, Schriftzügen oder (anderen)

Zeichen oder Symbolen versehen sein kann, wodurch sich Zuordnungen (ebenfalls) vereinfachen und/oder Zuordnungen schneller vorgenommen werden können.

In Ausgestaltung der vorliegenden Offenbarung ist der Hohlkörper ferner kapselförmig, d.h. als Kapsel, gestaltet.

Mit anderen Worten ist es erfindungsgemäß bevorzugt, wenn das Kennzeichnungselement eingekapselt vorliegt. Die Kapsel kann dabei einen eckenlosen, bevorzugt kreisförmigen, ovalen oder elliptischen, Querschnitt oder einen eckigen, d.h. polygonalen, insbesondere rechteckförmigen, wie beispielsweise quadratischen, Querschnitt aufweisen.

In weiterer Ausgestaltung der vorliegenden Offenbarung ist der Hohlkörper wenigstens abschnittsweise, insbesondere nur abschnittsweise oder durchgehend, hohlzylindrisch, insbesondere mit einem eckenlosen, bevorzugt kreisförmigen, ovalen oder elliptischen, Querschnitt oder mit einem eckigen, d.h. polygonalen, insbesondere rechteckförmigen, wie beispielsweise quadratischen, Querschnitt gestaltet. Besonders bevorzugt ist der Hohlkörper hohlkreiszylindrisch, d.h. in Form eines Hohlzylinders mit kreisförmigem Querschnitt, gestaltet.

Erfindungsgemäß ist der Hohlkörper mit dem darin enthaltenen Kennzeichnungselement nach Art oder in Form einer Libelle, bevorzugt nach Art oder in Form einer Röhrenlibelle, insbesondere einer Setzlibelle, oder einer Dosenlibelle, insbesondere mit oder ohne Markierungen auf einer Außenoberfläche der Libelle, gestaltet.

In weiterer Ausgestaltung der Erfindung besteht der Hohlkörper wenigstens abschnittsweise, insbesondere nur abschnittsweise oder vollständig, aus Glas, insbesondere einem blick- oder lichtdurchlässigen Glas, oder einem Kunststoff, insbesondere blick- oder lichtdurchlässigen Kunststoff. Bei dem Glas handelt es sich vorzugsweise um ein silikathaltiges Glas oder um ein aus Silikat bestehendes Glas. Der Kunststoff kann beispielsweise ausgewählt sein aus der Gruppe bestehend aus Polymethylmethacrylat und Mischungen davon.

In weiterer Ausgestaltung der Erfindung ist der Hohlkörper wenigstens abschnittsweise, insbesondere nur abschnittsweise oder vollständig, in einem Hohlkörpergehäuse, insbesondere in einem Libellengehäuse, enthalten oder eingebaut, oder wenigstens abschnittsweise, insbesondere nur abschnittsweise oder vollständig, in ein Hohlkörpergehäuse, insbesondere in ein Libellengehäuse, aufgenommen oder eingefügt. Dadurch kann mit besonderem Vorteil ein noch besserer Schutz des Kennzeichnungselements vor exogenen Einflüssen erzielt werden. Das Hohlkörpergehäuse, insbesondere Libellengehäuse, kann ferner wenigstens abschnittsweise, insbesondere nur abschnittsweise oder durchgehend, zylindrisch, insbesondere mit einem eckenlosen, wie beispielsweise kreisförmigen, ovalen oder elliptischen, Querschnitt oder mit einem eckigen, wie beispielsweise rechteckigen, insbesondere quadratischen, Querschnitt, gestaltet sein.

In weiterer Ausgestaltung der Erfindung ist der Hohlkörper wenigstens abschnittsweise, insbesondere nur abschnittsweise oder vollständig, in eine Aufnahmeaussparung eines Hohlkörpereinsatzes, insbesondere eines Libelleneinsatzes, eingefügt, insbesondere eingepresst.

Unter dem Ausdruck "Hohlkörpereinsatz" soll im Sinne der vorliegenden Erfindung ein Bauteil verstanden werden, das zusammen mit dem Hohlkörper (mit dem darin enthaltenen Kennzeichnungselement) in/an ein medizinisches Instrument, in/an ein Halbfabrikat eines medizinischen Instruments oder in/an ein Teil eines medizinischen Instruments eingebaut/montiert, insbesondere in eine Aufnahmeaussparung eines medizinischen Instruments, in/an ein Halbfabrikat eines medizinischen Instruments oder eines Teils eines medizinischen Instruments, eingefügt, insbesondere eingepresst, wird.

Entsprechend soll unter dem Ausdruck "Libelleneinsatz" im Sinne der vorliegenden Erfindung ein Bauteil verstanden werden, das zusammen mit der Libelle in/an ein medizinisches Instrument, in/an ein Halbfabrikat eines medizinischen Instruments oder in/an ein Teil eines medizinischen Instruments eingebaut/montiert, insbesondere in eine Aufnahmeaussparung eines medizinischen Instruments, in/an ein Halbfabrikat eines medizinischen Instruments oder eines Teils eines medizinischen Instruments, eingefügt, insbesondere eingepresst, wird.

Unter dem im Zusammenhang des Hohlkörpereinsatzes, insbesondere Libelleneinsatzes, verwendeten Ausdruck "Aufnahmeaussparung" soll im Sinne der vorliegenden Erfindung eine Aussparung des Hohlkörpereinsatzes, insbesondere Libelleneinsatzes, zum Aufnehmen des Hohlkörpers, insbesondere der Libelle, (mit dem darin enthaltenen Kennzeichnungselement) verstanden werden.

Der Hohlkörpereinsatz, insbesondere Liebelleneinsatz, kann ferner abschnittsweise, insbesondere nur abschnittsweise, zylindrisch, insbesondere mit einem eckenlosen, wie beispielsweise kreisförmigen, ovalen oder elliptischen, Querschnitt oder mit einem eckigen, wie beispielsweise rechteckigen, insbesondere quadratischen, Querschnitt, gestaltet sein.

In weiterer Ausgestaltung der Erfindung ist das Hohlkörpergehäuse, insbesondere Libellengehäuse, wenigstens abschnittsweise, insbesondere nur abschnittsweise oder vollständig, blick- oder lichtdurchlässig gestaltet. Bevorzugt besteht das Hohlkörpergehäuse hierzu wenigstens abschnittsweise, insbesondere nur abschnittsweise oder vollständig, aus Glas, d.h. einem blick- oder lichtdurchlässigem Glas, oder einem blick- oder lichtdurchlässigen Kunststoff. Der blick- oder lichtdurchlässige Kunststoff kann beispielsweise Polymethylmethacrylat sein.

In weiterer Ausgestaltung der Erfindung besteht der Hohlkörpereinsatz, insbesondere Libelleneinsatz, aus einem Kunststoff, wie beispielsweise Polyolefine, Polyethylen, Polypropylen, Polyvinylidenfluorid, Polytetrafluorethylen, Polyetherketon, Polyetheretherketon, Polyester, Polyethylenterephthalat, Polypropylenterephthalat, Polybutylenterephthalat, Polyamide und Mischungen davon, und/oder einem Metall und/oder einer Metalllegierung, wie beispielsweise Edelstahl.

In weiterer Ausgestaltung der Erfindung ist der Hohlkörper (mit dem darin enthaltenen Kennzeichnungselement), insbesondere zusammen mit dem Hohlkörpergehäuse oder dem Hohlkörpereinsatz, in eine Aufnahmeaussparung des medizinischen Instruments oder des Teils eines medizinischen Instruments eingefügt, insbesondere eingepresst.

Unter dem im Zusammenhang des medizinischen Instruments oder des Teils eines medizinischen Instruments verwendeten Ausdruck "Aufnahmeaussparung" soll im Sinne der vorliegenden Erfindung eine Aussparung des medizinischen Instruments oder des Teils eines medizinischen Instruments zum Aufnehmen des Hohlkörpers, insbesondere zusammen mit dem Hohlkörpergehäuse, verstanden werden. Bevorzugt soll unter dem im Zusammenhang des medizinischen Instruments oder des Teils eines medizinischen Instruments verwendeten Ausdruck "Aufnahmeaussparung" im Sinne der vorliegenden Erfindung eine Aussparung des medizinischen Instruments oder des Teils eines medizinischen Instruments zum Aufnehmen der bereits erwähnten Libelle, insbesondere zusammen mit dem bereits erwähnten Libellengehäuse, verstanden werden.

Bevorzugt ist das Kennzeichnungselement sterilisationsbeständig und/oder säurebeständig und/oder biokompatibel ausgebildet.

In weiterer Ausgestaltung der vorliegenden Offenbarung handelt es sich bei dem Kennzeichnungselement um eine Flüssigkeit, die wenigstens ein Farbmittel aufweist. Die Flüssigkeit kann in Form einer Dispersion, Lösung oder Suspension, vorzugsweise in Form einer Lösung, vorliegen. Die Flüssigkeit kann ferner neben dem wenigstens einen Farbmittel ein Dispersions-, Suspensions- oder Lösungsmittel aufweisen, das vorzugsweise ausgewählt ist aus der Gruppe bestehend aus Wasser, Ethanol, Isopropanol und Mischungen davon.

Die das wenigstens eine Farbmittel aufweisende Flüssigkeit kann ferner eine Luft- oder Gasblase aufweisen. Die Luft- oder Gasblase kann mit besonderem Vorteil als Orientierungshilfe zur Ausrichtung, insbesondere horizontalen oder vertikalen Ausrichtung, des medizinischen Instruments oder des Teils eines medizinischen Instruments dienen.

Erfindungsgemäß handelt es sich bei dem Kennzeichnungselement um einen Feststoff, der wenigstens ein Farbmittel aufweist.

In weiterer Ausgestaltung der Erfindung liegt das Kennzeichnungselement, insbesondere der das wenigstens eine Farbmittel aufweisende Feststoff, in Form von farbigen oder gefärbten Partikeln vor. Vorzugsweise liegt der das wenigstens eine Farbmittel aufweisende Feststoff als farbiges oder gefärbtes Pulver oder als farbiges oder gefärbtes Granulat vor. Insbesondere kann es sich bei dem Kennzeichnungselement, insbesondere bei dem das wenigstens eine Farbmittel aufweisenden Feststoff, um ein Kunststoffpulver oder Kunststoffgranulat handeln, das wenigstens ein Farbmittel aufweist (sogenanntes farbmittelhaltiges Compound). Der Kunststoff kann dabei ausgewählt sein aus der Gruppe bestehend aus Polyolefine, Polyethylen, Polypropylen, Polyvinylidenfluorid, Polytetrafluorethylen, Polyetherketon, Polyetheretherketon, Polyester, Polyethylenterephthalat, Polypropylenterephthalat, Polybutylenterephthalat, Polyamide und Mischungen davon.

Alternativ kann es sich bei dem Kennzeichnungselement, insbesondere bei dem das wenigstens eine Farbmittel aufweisenden Feststoff, um einen mit wenigstens einem Farbmittel beladenen Träger handeln. Beispielsweise kann das Kennzeichnungselement, insbesondere der das wenigstens eine Farbmittel aufweisende Feststoff, in Form von mit wenigstens einem Farbmittel beladenen Trägerpartikeln vorliegen.

In weiterer Ausgestaltung der Erfindung handelt es sich bei dem Kennzeichnungselement, insbesondere bei dem das wenigstens eine Farbmittel aufweisenden Feststoff, um ein farbiges, bemaltes, lackiertes (insbesondere farbig lackiertes) oder bedrucktes (insbesondere farbig bedrucktes) Glas, insbesondere um ein farbiges, bemaltes, lackiertes (insbesondere farbig lackiertes) oder bedrucktes (insbesondere farbig bedrucktes) Hinterglas.

Unter dem Ausdruck "farbiges Glas" soll im Sinne der vorliegenden Erfindung ein Glas verstanden werden, das eine andere Farbe als Weiß oder Schwarz aufweist (sogenanntes Buntfarbenglas).

Unter dem Ausdruck "farbiges Hinterglas" soll im Sinne der vorliegenden Erfindung ein Glas verstanden werden, das lediglich auf einer Seite oder Seitenfläche, insbesondere lediglich auf einer dem Betrachter abgewandten Seite (Rückseite) oder Seitenfläche (Rückseitenfläche), farbig gestaltet ist, d.h. eine andere Farbe als Weiß oder Schwarz aufweist.

Unter dem Ausdruck "bemaltes Hinterglas" soll im Sinne der vorliegenden Erfindung ein Glas verstanden werden, das lediglich auf einer Seite oder Seitenfläche, insbesondere lediglich auf einer dem Betrachter abgewandten Seite (Rückseite) oder Seitenfläche (Rückseitenfläche), bemalt ist.

Unter dem Ausdruck "lackiertes Hinterglas" soll im Sinne der vorliegenden Erfindung ein Glas verstanden werden, das lediglich auf einer Seite oder Seitenfläche, insbesondere lediglich auf einer dem Betrachter abgewandten Seite (Rückseite) oder Seitenfläche (Rückseitenfläche), lackiert, insbesondere farbig lackiert, ist.

Unter dem Ausdruck "bedrucktes Hinterglas" soll im Sinne der vorliegenden Erfindung ein Glas verstanden werden, das lediglich auf einer Seite oder Seitenfläche, insbesondere lediglich auf einer dem Betrachter abgewandten Seite (Rückseite) oder Seitenfläche (Rückseitenfläche), bedruckt, insbesondere farbig bedruckt, ist.

Das farbige Glas kann ausgewählt sein aus der Gruppe bestehend aus lila gefärbtes Glas, blau gefärbtes Glas, grün gefärbtes Glas, gelb gefärbtes Glas, orange gefärbtes Glas und rot gefärbtes Glas. Entsprechend kann das farbige Hinterglas ausgewählt sein aus der Gruppe bestehend aus lila gefärbtes Hinterglas, blau gefärbtes Hinterglas, grün gefärbtes Hinterglas, gelb gefärbtes Hinterglas, orange gefärbes Hinterglas und rot gefärbtes Hinterglas.Bevorzugt ist das vorher erwähnte wenigstens eine Farbmittel sterilisationsbeständig und/oder säurebeständig und/oder biokompatibel ausgebildet.

In weiterer Ausgestaltung der Erfindung handelt es sich bei dem wenigstens einen Farbmittel um wenigstens einen Farbstoff. Der wenigstens eine Farbstoff ist vorzugsweise ausgewählt aus der Gruppe bestehend aus Anthrachinonfarbstoffe, Azofarbstoffe, Dioxazinfarbstoffe, indigoide Farbstoffe, Metallkomplexfarbstoffe, Formazanfarbstoffe, Phthalocyaninfarbstoffe, Methinfarbstoffe, Direktfarbstoffe, Dispersionsfarbstoffe, Entwicklungs- oder Kupplungsfarbstoffe, kationische Farbstoffe, Küpenfarbstoffe, Beizenfarbstoffe, Lösungsmittelfarbstoffe, Reaktivfarbstoffe, Säurefarbstoffe, funktionelle Farbstoffe, Lebensmittelfarbstoffe, Vitalfarbstoffe, Anilinblau, Azokarmin wie Azokarmin G (Rosindulin), Bromcresolgrün, Coomassie-Brillant-Blau, Eisenhämatoxylin, Eosin, Fuchsin, Hämatoxylin, Kongorot, Lichtgrün, Methylenblau, Methylorange, Methylrot und Mischungen davon.

Die Anthrachinonfarbstoffe können insbesondere ausgewählt sein aus der Gruppe bestehend aus C.I. Vat Blue 4, C.I. Disperse Blue 87, C.I. Acid Blue 25, C.I. Reactive Blue 19 und Mischungen davon.

Die Azofarbstoffe können insbesondere ausgewählt sein aus der Gruppe bestehend aus C.I. Reactive Black 5, C.I. Disperse Yellow 241, C.I. Mordant Black 9, C.I. Solvent Yellow 19 und Mischungen davon.

Die Dioxazinfarbstoffe können insbesondere ausgewählt sein aus der Gruppe bestehend aus C.I. Direct Blue 106, C.I. Reactive Blue 204 und Mischungen davon.

Die indigoiden Farbstoffe können insbesondere ausgewählt sein aus der Gruppe bestehend aus C.I. Vat Blue 1 (Indigo), C.I. Natural Violet 1, Indirubin und Mischungen davon.

Die Formazanfarbstoffe können insbesondere ausgewählt sein aus der Gruppe bestehend aus C.I. Reactive Blue 160, C.I. Reactive Blue 235 und Mischungen davon.

Die Phthalocyaninfarbstoffe können insbesondere ausgewählt sein aus der Gruppe bestehend aus Phthalocyanin, C.I. Reactive Blue 7 und Mischungen davon.

Die Methinfarbstoffe können insbesondere ausgewählt sein aus der Gruppe bestehend aus C.I. Basic Red 22, C.I. Disperse Yellow 31, C.I. Basic Green 4 (Malachitgrün) und Mischungen davon.

Die Beizenfarbstoffe können insbesondere ausgewählt sein aus der Gruppe bestehend aus C.I. Mordant Black 9, C.I. Mordant Yellow 8, C.I. Mordant Black 7, C.I. Mordant Red 60, C.I. Mordant Blue 9 und Mischungen davon.

Die Direktfarbstoffe können insbesondere ausgewählt sein aus der Gruppe bestehend aus C.I. Direct Blue 8, C.I. Direct Orange 26, C.I. Direct Yellow 9 und Mischungen davon.

Die Dispersionsfarbstoffe können insbesondere ausgewählt sein aus der Gruppe bestehend aus C.I. Disperse Orange 44, C.I. Disperse Blue 797, C.I. Disperse Red 177 und Mischungen davon.

Die Entwicklungs- oder Kupplungsfarbstoffe können insbesondere ausgewählt sein aus der Gruppe bestehend aus C.I. Azoic Coupling Component 2 (Naphthol AS), C.I. Azoic Coupling Component 35 (Naphtol AS-LG), C.I. Azoic Diazo Component 3 (Echtscharlachsalz GG), C.I. Azoic Diazo Component 35 (Variaminblausalz B) und Mischungen davon.

Die kationischen Farbstoffe können insbesondere ausgewählt sein aus der Gruppe bestehend aus C.I. Basic Orange 22, C.I. Basic Blue 3, C.I. Basic Blue 54, C.I. Basic Red 18 und Mischungen davon.

Die Küpenfarbstoffe können insbesondere ausgewählt sein aus der Gruppe bestehend aus C.I. Vat Green 11, C.I. Vat Orange 7, C.I. Vat Red 23, C.I. Vat Yellow 1 (Flavanthron) und Mischungen davon.

Die Lösungsmittelfarbstoffe können insbesondere ausgewählt sein aus der Gruppe bestehend aus C.I. Solvent Yellow 124, C.I. Solvent Red 27, C.I. Solvent Blue 35, C.I. Solvent Yellow 32, C.I. Solvent Black 3, C.I. Solvent Red 8 und Mischungen davon.

Die Reaktivfarbstoffe können insbesondere ausgewählt sein aus der Gruppe bestehend aus C.I. Reactive Orange 107, C.I. Reactive Blue 2, C.I. Reactive Blue 21, C.I. Reactive Red 227, C.I. Reactive Orange 1 und Mischungen davon.

Die Säurefarbstoffe können insbesondere ausgewählt sein aus der Gruppe bestehend aus C.I. Acid Black 1, C.I. Acid Yellow 36, C.I. Acid Blue 117, C.I. Acid Orange 19, C.I. Acid Blue 3 (Patentblau V) und Mischungen davon.

Die Lebensmittelfarbstoffe können insbesondere ausgewählt sein aus der Gruppe bestehend aus Carotinoide, Beerenfarbstoffe (Anthocyane), Beten-Farbstoffen (Betanin), Farbstoffe von Gewürzen wie Paprika, Safran und Gelbwurzel (Curcumin), Azofarbstoffe wie Tartrazin (E 102), Gelborange S (E 110), Azorubin (E 122), Cochenillerot A (E 124), Allurarot AC (E 129), Nichtazofarbstoffe wie Chinolingelb (E 104), Indigotin (E 132), Grün S (E 142), Amaranth (E 123), Anthocyane (E 163), Betanin (E 162), Braun FK (E 154), Braun HT (E 155), Brilliantblau FCF (E 133), Brilliantschwarz BN (E 151), Canthaxanthin (E 161 g), Carotin ( E160 a), Annatto (E 160 b), Capsanthin (E 160 c), Lycopin (E 160 d), 8'-Apo-beta-Caroten-8'-al (E 160 e), Ethyl-8'-apo-beta-caroten-8'-oat (E 160 f), Chlorophyll (E 140), Curcumin (E 100), Erythrosin (E 127), Indigokarmin (E 132), Koschenille (E 120), kupferhaltige Komplexe der Chlorophylle und Chlorophylline (E 141), Litholrubin BK (E 180), Lutein (E 161 b), Patentblau V (E 131), Riboflavin (E 101), Riboflavin-5-Phosphat(E 101a), Tartrazin, Zuckerkulör (E 150 a), Sulfitlaugen-Zuckerkulör (E 150 b), Ammoniak-Zuckerkulör (E 150 c), Ammonsulfit-Zuckerkulör (E 150 d) und Mischungen davon.

In weiterer Ausgestaltung der Erfindung handelt es sich bei dem wenigstens einen Farbmittel um wenigstens ein Pigment. Bevorzugt ist das wenigstens eine Pigment ausgewählt aus der Gruppe bestehend aus Titandioxid, Aluminium, Silber, Gold, Calciumcarbonat, Titandioxid, Eisenoxid, Pflanzenkohle und Mischungen davon.

In weiterer Ausgestaltung der Erfindung handelt es sich bei dem Kennzeichnungselement um ein Logo (d.h. ein grafisch gestaltetes Zeichen), ein Bild, eine Zeichnung, einen Schriftzug (wie beispielsweise ein Slogan) oder ein Symbol. Insbesondere kann es sich bei dem Logo um ein farbiges oder gefärbtes Logo, insbesondere aufweisend wenigstens ein der vorgenannten Farbmittel, handeln. Weiter kann es sich bei dem Bild insbesondere um ein farbiges oder gefärbtes Bild, insbesondere aufweisend wenigstens ein der vorgenannten Farbmittel, handeln. Weiter kann es sich bei der Zeichnung insbesondere um eine farbige oder gefärbte Zeichnung, insbesondere aufweisend wenigstens ein der vorgenannten Farbmittel, handeln. Weiter kann es sich bei dem Schriftzug insbesondere um einen farbigen oder gefärbten Schriftzug, insbesondere aufweisend wenigstens ein der vorgenannten Farbmittel, handeln. Weiter kann es sich bei dem Symbol insbesondere um ein farbiges oder gefärbtes Symbol, insbesondere aufweisend wenigstens ein der vorgenannten Farbmittel, handeln.

Bevorzugt handelt es sich bei dem medizinischen Instrument um ein chirurgisches Instrument, insbesondere um ein chirurgisches Instrument für die Hüft-, Knie- oder Wirbelsäulenchirurgie. Vorzugsweise handelt es sich bei dem medizinischen Instrument um eine chirurgische Säge. Die chirurgische Säge kann als Handsäge, Drahtsäge oder oszillierende Säge, insbesondere elektrisch oder pneumatisch angetriebene oszillierende Säge, gestaltet sein. Bei dem Teil eines medizinischen Instruments handelt es sich bevorzugt um ein Teil eines chirurgischen Instruments, insbesondere um ein Teil eines chirurgischen Instruments für die Hüft-, Knie- oder Wirbelsäulenchirurgie. Vorzugsweise handelt es sich bei dem Teil eines medizinischen Instruments um einen chirurgischen Sägeblock oder ein chirurgisches Sägeblatt.

Die Erfindung betrifft ferner ein Kit oder Set. Das Kit oder Set weist räumlich oder physikalisch getrennt voneinander eine Vielzahl von medizinischen Instrumenten, d.h. zwei oder mehr, beispielsweise drei, vier, fünf oder sechs, medizinische Instrumente, oder eine Vielzahl von Teilen von medizinischen Instrumenten, d.h. zwei oder mehr, beispielsweise drei, vier, fünf oder sechs, Teile von medizinischen Instrumenten, gemäß der vorliegenden Erfindung auf. Die medizinischen Instrumente oder die Teile von medizinischen Instrumenten sind bevorzugt unterschiedlich gestaltet. Besonders bevorzugt unterscheiden sich die medizinischen Instrumente oder die Teile von medizinischen Instrumenten, insbesondere nur, in Bezug auf ihre Größe, d.h. in Bezug auf wenigstens eine Abmessung, insbesondere Länge und/oder Breite und/oder Höhe und/oder Durchmesser, und das Kennzeichnungselement voneinander. Vorzugsweise unterscheiden sich die Kennzeichnungselemente der medizinischen Instrumente oder der Teile von medizinischen Instrumenten, insbesondere nur, in Bezug auf ein Farbmittel und/oder einen Farbmittelanteil voneinander. Insbesondere kann es sich bei den Kennzeichnungselementen der medizinischen Instrumente oder der Teile von medizinischen Instrumenten um Flüssigkeiten, insbesondere Dispersionen, Lösungen oder Suspensionen, handeln, die jeweils wenigstens ein Farbmittel aufweisen, wobei sich die Flüssigkeiten, insbesondere Dispersionen, Lösungen oder Suspensionen, in Bezug auf das wenigstens eine Farbmittel und/oder den Anteil (die Konzentration) des wenigstens einen Farbmittels voneinander unterscheiden. Mit anderen Worten kann es sich bei den Kennzeichnungselementen der medizinischen Instrumente oder der Teile von medizinischen Instrumenten insbesondere um unterschiedlich gefärbte Flüssigkeiten, insbesondere Dispersionen, Lösungen oder Suspensionen, handeln.

Alternativ kann es sich bei den Kennzeichnungselementen der medizinischen Instrumente oder der Teile von medizinischen Instrumenten um Feststoffe, insbesondere partikuläre Feststoffe, beispielsweise Pulver oder Granulate, handeln, die jeweils wenigstens ein Farbmittel aufweisen, wobei die Farbmittel der Feststoffe, insbesondere partikulären Feststoffe, beispielsweise Pulver oder Granulate, unterschiedlich sind. Mit anderen Worten kann es sich bei den Kennzeichnungselementen der medizinischen Instrumente oder der Teile von medizinischen Instrumenten insbesondere um unterschiedlich gefärbte Feststoffe, insbesondere partikuläre Feststoffe, wie beispielsweise Pulver oder Granulate, handeln.

Alternativ kann es sich bei den Kennzeichnungselementen der medizinischen Instrumente oder der Teile von medizinischen Instrumenten um farbige, bemalte oder lackierte Gläser, insbesondere Hintergläser, handeln, wobei sich die Gläser, insbesondere Hintergläser, in Bezug auf ihre Farbe oder Färbung voneinander unterscheiden. Mit anderen Worten kann es sich bei den Kennzeichnungselementen der medizinischen Instrumente oder der Teile von medizinischen Instrumenten um unterschiedlich farbige oder gefärbte, unterschiedlich bemalte oder unterschiedlich (farbig) lackierte Gläser, insbesondere Hintergläser, handeln.

Bevorzugt handelt es sich bei den medizinischen Instrumenten um chirurgische Instrumente, insbesondere für die Hüft-, Knie- oder Wirbelsäulenchirurgie. Vorzugsweise handelt es sich bei den medizinischen Instrumenten um chirurgische Sägen. Die chirurgischen Sägen können insbesondere als Handsägen, Drahtsägen oder oszillierende Sägen, insbesondere elektrisch oder pneumatisch angetriebene oszillierende Sägen, gestaltet sein.

Bei den Teilen von medizinischen Instrumenten handelt es sich bevorzugt um Teile von chirurgischen Instrumenten, insbesondere für die Hüft-, Knie- oder Wirbelsäulenchirurgie. Vorzugsweise handelt es sich bei den Teilen von chirurgischen Instrumenten um chirurgische Sägeblätter oder chirurgische Sägeblöcke.

Durch das erfindungsgemäße Kit oder Set wird insbesondere die Erkennbarkeit/Unterscheidbarkeit und/oder die Zuordnung der medizinischen Instrumente oder der Teile von medizinischen Instrumenten für Anwender, insbesondere Ärzte, OP-Personal sowie Mitarbeiter in zentralen Sterilgutversorgungsabteilungen, deutlich erleichtert. Dadurch lassen sich OP-Zeiten verkürzen und die Sicherheit für Patienten zusätzlich erhöhen. Ferner sind hierdurch in zentralen Sterilgutversorgungsabteilungen deutlich kürzere Durchlaufzeiten realisierbar, da beispielsweise die Zuordnung von medizinischen Instrumenten oder medizinischen Instrumententeilen in zugehörige Siebe signifikant vereinfacht werden kann.

Bezüglich weiterer Merkmale und Vorteile des Kits bzw. Sets, insbesondere bezüglich der medizinischen Instrumente oder Teile von medizinischen Instrumenten sowie der Kennzeichnungselemente, wird vollständig auf die bisherige Beschreibung Bezug genommen. Die dort insbesondere im Zusammenhang der medizinischen Instrumente oder Teile von medizinischen Instrumenten sowie der Kennzeichnungselemente beschriebenen Merkmale und Vorteile gelten entsprechend auch für das erfindungsgemäße Kit oder Set.

Die Erfindung betrifft ferner ein weiteres Kit oder Set, aufweisend räumlich oder physikalisch getrennt voneinander eine Anzahl, insbesondere Vielzahl, von medizinischen Instrumenten oder eine Anzahl, insbesondere Vielzahl, von Teilen von medizinischen Instrumenten und eine Anzahl, insbesondere Vielzahl, von Kennzeichnungselementen, bevorzugt eine der Anzahl, insbesondere Vielzahl, von medizinischen Instrumenten entsprechende Anzahl, insbesondere Vielzahl, von Kennzeichnungselementen.

Die Kennzeichnungselemente können gleich oder verschieden ausgebildet sein. Vorzugsweise sind die Kennzeichnungselemente ausgewählt aus der Gruppe bestehend aus
- Flüssigkeit, die wenigstens ein Farbmittel aufweist,
- Feststoff, der wenigstens ein Farbmittel aufweist,
- farbiges, bemaltes, lackiertes oder bedrucktes Glas, insbesondere Hinterglases,
- Logo,
- Bild oder Zeichnung,
- Schriftzug,
- Symbol und
- Kombinationen davon.

Bei den medizinischen Instrumenten handelt es sich vorzugsweise um chirurgische Instrumente, insbesondere um chirurgische Instrumente für die Hüft-, Knie- oder Wirbelsäulenchirurgie. Vorzugsweise handelt es sich bei den medizinischen Instrumenten um chirurgische Sägen. Die chirurgischen Sägen können als Handsägen, Drahtsägen oder oszillierende Sägen, insbesondere elektrisch oder pneumatisch angetriebene oszillierende Sägen, gestaltet sein. Bei den Teilen von medizinischen Instrumenten handelt es sich bevorzugt um Teile von chirurgischen Instrumenten, insbesondere für die Hüft-, Knie- oder Wirbelsäulenchirurgie. Vorzugsweise handelt es sich bei den Teilen von medizinischen Instrumenten um chirurgische Sägeblöcke oder chirurgische Sägeblätter.

Bezüglich weiterer Merkmale und Vorteile des weiteren Kit bzw. Sets, insbesondere bezüglich der medizinischen Instrumente oder Teile von medizinischen Instrumenten sowie der Kennzeichnungselemente, wird vollständig auf die bisherige Beschreibung Bezug genommen. Die dort insbesondere im Zusammenhang der medizinischen Instrumente oder Teile von medizinischen Instrumenten sowie der Kennzeichnungselemente beschriebenen Merkmale und Vorteile gelten entsprechend auch für das weitere erfindungsgemäße Kit oder Set.

Weitere Merkmale und Vorteile der Erfindung ergeben sich aus den Ansprüchen sowie aus der nachfolgenden Beschreibung bevorzugter Ausführungsbeispiele der Erfindung, die anhand der Zeichnungen dargestellt sind.

### FIGURENKURZBESCHREIBUNGEN

- Fig. 1: zeigt eine schematische Perspektivdarstellung einer Ausführungsform eines erfindungsgemäßen Instruments,
- Fig. 2: zeigt schematisch eine vergrößerte Detaildarstellung eines geschlossenen Hohlkörpers mit gefärbter Flüssigkeit und zugehörigem Hohlkörpergehäuse eines erfindungsgemäßen Instruments nach Fig. 1,
- Fig. 3: zeigt eine schematische Perspektivdarstellung einer weiteren Ausführungsform eines erfindungsgemäßen Instruments,
- Fig. 4: zeigt eine schematische Längsschnittdarstellung eines erfindungsgemäßen Instruments entlang einer Schnittlinie IV-IV nach Fig. 3,
- Fig. 5: zeigt eine schematische Perspektivdarstellung einer weiteren Ausführungsform eines erfindungsgemäßen Instruments und
- Fig. 6: zeigt eine schematische Querschnittsdarstellung eines erfindungsgemäßen Instruments entlang einer Schnittlinie VI-VI nach Fig. 5.

### AUSFÜHRLICHE FIGURENBESCHREIBUNGEN

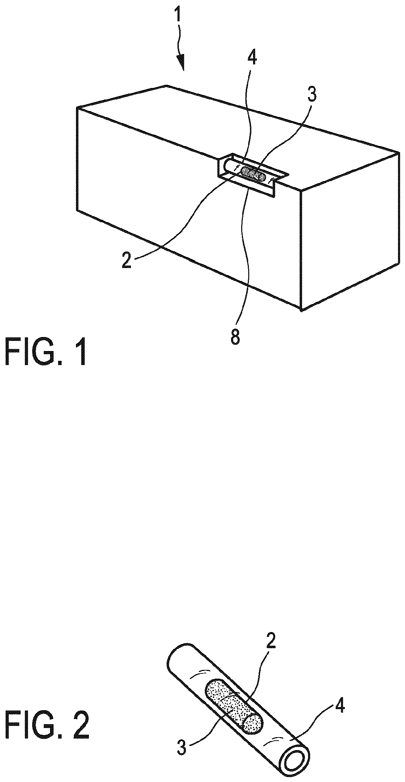

Fig. 1 zeigt schematisch eine Perspektivdarstellung eines medizinischen Instruments 1 gemäß der vorliegenden Erfindung.

Das medizinische Instrument 1 weist einen geschlossenen, insbesondere hermetisch geschlossenen, Hohlkörper 2 auf. Der Hohlkörper 2 ist wenigstens abschnittsweise, vorzugsweise nur abschnittsweise oder vollständig, blick- oder lichtdurchlässig gestaltet. Der Hohlkörper 2 besitzt bevorzugt die Form einer Kapsel, insbesondere mit rechteckigem, z.B. quadratischem, oder kreisförmigem Querschnitt. Besonders bevorzugt ist der Hohlkörper 2 wenigstens abschnittsweise hohlkreiszylindrisch gestaltet, d.h. in Form eines Hohlzylinders mit einem kreisförmigen Querschnitt.

Weiter bevorzugt besteht der Hohlkörper 2 wenigstens abschnittsweise, insbesondere nur abschnittsweise oder vollständig, aus einem lichtdurchlässigen Glas oder einem lichtdurchlässigen Kunststoff, wie beispielsweise Polymethylmethacrylat.

In dem Hohlkörper 2 ist ein Kennzeichnungselement 3 enthalten, das durch den wenigstens abschnittsweise blick- oder lichtdurchlässigen Hohlkörper 2 hindurch einseh- oder erkennbar ist.

Das Kennzeichnungselement 3 kann zum Beispiel als Flüssigkeit, vorzugsweise Lösung, ausgebildet sein, die wenigstens ein Farbmittel aufweist. Vorzugsweise ist das wenigstens eine Farbmittel sterilisationsbeständig und/oder säurebeständig und/oder biokompatibel. Bei dem wenigstens einen Farbmittel kann es sich insbesondere um wenigstens einen Farbstoff oder um wenigstens ein Pigment handeln. Bezüglich geeigneter Farbstoffe oder Pigmente sei auf die allgemeine Beschreibung verwiesen.

Der Hohlkörper 2 mit dem darin enthaltenen Kennzeichnungselement 3 ist vorzugsweise als Röhrenlibelle gestaltet. Ferner kann eine Außenoberfläche, insbesondere eine beim bestimmungsgemäßen Gebrauch des medizinischen Instruments 1 sichtbare Außenoberfläche, des Hohlkörpers 2 mit einer Markierung, wie beispielsweise einer Skala oder Ähnlichem, versehen sein. Dadurch kann die Erkennbarkeit und/oder Unterscheidbarkeit des medizinischen Instruments 1 gegenüber anderen Instrumenten zusätzlich verbessert werden.

Der Hohlkörper 2 mit dem darin enthaltenen Kennzeichnungselement 3 ist ferner in ein Hohlkörpergehäuse, insbesondere Libellengehäuse, 4 aufgenommen oder eingefügt, insbesondere eingepresst.

Das Hohlkörpergehäuse, insbesondere Libellengehäuse, 4 kann dabei wenigstens abschnittsweise, insbesondere nur abschnittsweise oder vollständig, aus Glas oder Kunststoff, insbesondere aus einem blick- oder lichtdurchlässigen Glas oder blick- oder lichtdurchlässigen Kunststoff, bestehen.

Der Hohlkörper 2 ist ferner zusammen mit dem Hohlkörpergehäuse, insbesondere Libellengehäuse, 4 in eine Aufnahmeaussparung 8 des medizinischen Instruments 1 eingefügt, insbesondere eingepresst.

Fig. 2 zeigt schematisch eine vergrößerte Detaildarstellung eines geschlossenen Hohlkörpers 2 mit einem darin enthaltenen Kennzeichnungselement 3 sowie einem zugehörigen Hohlkörpergehäuse 4 nach Fig. 1 als zusammengebautes Bauteil zur weiteren Montage in/an ein medizinisches Instrument.

Bezüglich weiterer Merkmale und Vorteile des Hohlkörpers 2, dem darin enthaltenen Kennzeichnungselement 3 sowie des Hohlkörpergehäuses 4 wird vollständig auf die Figurenbeschreibung zur Fig. 1 Bezug genommen.

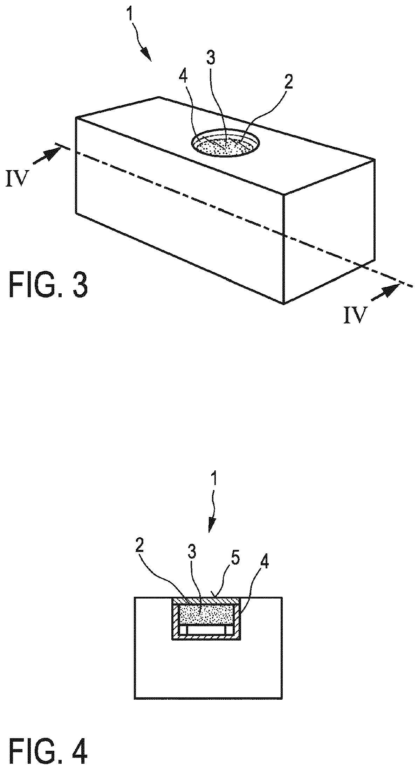

Fig. 3 zeigt schematisch eine weitere Perspektivdarstellung eines medizinischen Instruments 1 gemäß der vorliegenden Erfindung.

Das medizinische Instrument 1 weist einen geschlossenen, insbesondere hermetisch geschlossenen, Hohlkörper 2 mit einem darin enthaltenen Kennzeichnungselement 3 auf. Der Hohlkörper 2 ist wenigstens abschnittsweise, vorzugsweise nur abschnittsweise oder vollständig, blick- oder lichtdurchlässig gestaltet, wobei das Kennzeichnungselement 3 durch den wenigstens abschnittsweise blick- oder lichtdurchlässig gestalteten Hohlkörper 2 hindurch einseh- oder erkennbar ist.

Das Kennzeichnungselement 3 kann beispielsweise als ein mit wenigstens einem Farbmittel gefärbter Feststoff, insbesondere als ein Kunststoffpulver, das wenigstens ein Farbmittel, insbesondere wenigstens einen Farbstoff oder wenigstens ein Pigment, aufweist, vorliegen. Bezüglich geeigneter Farbmittel, insbesondere Farbstoffe oder Pigmente, sei ebenfalls auf die allgemeine Beschreibung verwiesen.

Der Hohlkörper 2 mit dem darin enthaltenen Kennzeichnungselement 3 ist bevorzugt libellenförmig, insbesondere nach Art oder in Form einer sogenannten Dosenlibelle, gestaltet.

Der Hohlkörper 2 mit dem darin enthaltenen Kennzeichnungselement 3 ist ferner in ein Hohlkörpergehäuse, insbesondere Libellengehäuse, 4 aufgenommen oder eingefügt, insbesondere eingepresst.

Zweckmäßigerweise ist wenigstens ein beim bestimmungsgemäßen Gebrauch des medizinischen Instruments 1 sichtbarer Abschnitt 5 des Hohlkörpergehäuses, insbesondere Libellengehäuses, 4 aus einem blick- oder lichtdurchlässigen Material, insbesondere aus einem blick- oder lichtdurchlässigen Glas oder blick- oder lichtdurchlässigen Kunststoff, wie beispielsweise Polymethylmethacrylat, gefertigt, siehe Fig. 4.

Bezüglich weiterer Merkmale und Vorteile des in den Fig. 3 und 4 beschriebenen medizinischen Instruments 1 wird vollständig auf die Figurenbeschreibungen zu den Fig. 1 und 2 Bezug genommen. Die dort zusätzlich beschriebenen Merkmale und Vorteile können sinngemäß auch für das in den Fig. 3 und 4 beschriebene medizinische Instrument 1 gelten.

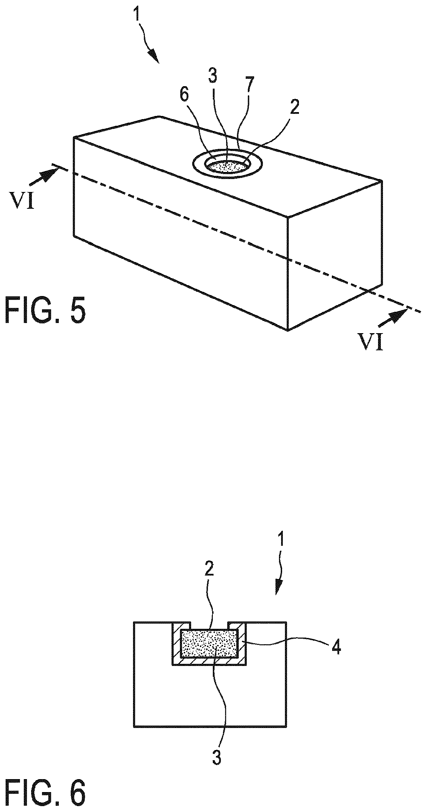

Die Fig. 5 und 6 zeigen schematisch eine weitere Ausführungsform eines medizinischen Instruments 1 gemäß der vorliegenden Erfindung.

Das medizinische Instrument 1 weist einen geschlossenen, insbesondere hermetisch geschlossenen, Hohlkörper 2 mit einem darin enthaltenen Kennzeichnungselement 3 auf. Der Hohlkörper 2 ist wenigstens abschnittsweise, vorzugsweise nur abschnittsweise oder vollständig, blick- oder lichtdurchlässig gestaltet, wobei das Kennzeichnungselement 3 durch den wenigstens abschnittsweise blick- oder lichtdurchlässig gestalteten Hohlkörper 2 hindurch einseh- oder erkennbar ist.

Das Kennzeichnungselement 3 kann beispielsweise ein Buntglas, insbesondere ein farbiges Hinterglas, sein. Alternativ kann es sich bei dem Kennzeichnungselement 3 um ein bemaltes, lackiertes oder bedrucktes Glas, insbesondere um ein bemaltes, lackiertes oder bedrucktes Hinterglas, handeln.

Der Hohlkörper 2 mit dem darin enthaltenen Kennzeichnungselement 3 ist bevorzugt libellenförmig, insbesondere nach Art oder in Form einer sogenannten Dosenlibelle, gestaltet.

Der Hohlkörper 2 mit dem darin enthaltenen Kennzeichnungselement 3 ist in eine Aufnahmeaussparung 6 eines Hohlkörpereinsatzes, insbesondere Libelleneinsatzes, 7 eingefügt, insbesondere eingepresst.

Bevorzugt ist der Hohlkörpereinsatz, insbesondere Libelleneinsatz, 7 einseitig offen, beispielsweise schalen- oder wannenförmig, gestaltet. Insbesondere bevorzugt ist der Hohlkörpereinsatz, insbesondere Libelleneinsatz, 7 auf einer beim bestimmungsgemäßen Gebrauch des medizinischen Instruments 1 sichtbaren Seite des Hohlkörpereinsatzes, insbesondere Libelleneinsatzes, 7 offen gestaltet ist, siehe Fig. 6.

Ferner ist der Hohlkörpereinsatz, insbesondere Libelleneinsatz, 7 bevorzugt aus einem blick- oder lichtdurchlässigen Material, insbesondere aus einem blick- oder lichtdurchlässigen Glas oder blick- oder lichtdurchlässigen Kunststoff, wie beispielsweise Polymethylmethacrylat, gefertigt.

Bezüglich weiterer Merkmale und Vorteile des in den Fig. 5 und 6 beschriebenen medizinischen Instruments 1 wird vollständig auf die vorangegangenen Figurenbeschreibungen Bezug genommen. Die dort zusätzlich beschriebenen Merkmale und Vorteile können sinngemäß auch für das in den Fig. 5 und 6 beschriebene medizinische Instrument 1 gelten.

Bei dem in den vorangegangenen Figurenbeschreibungen beschriebenen medizinischen Instrument 1 kann es sich insbesondere um ein chirurgisches Instrument, vorzugsweise für die Hüft-, Knie- oder Wirbelsäulenchirurgie, handeln.

## Patentansprüche

1. Medizinisches Instrument (1) oder Teil eines medizinischen Instruments (1), wobei das medizinische Instrument bzw. das Teil eines medizinischen Instruments (1) einen geschlossenen Hohlkörper (2) aufweist, der wenigstens abschnittsweise lichtdurchlässig gestaltet ist, wobei der Hohlkörper (2) ein Kennzeichnungselement (3) enthält, wobei das Kennzeichnungselement (3) durch den wenigstens abschnittsweise lichtdurchlässig gestalteten Hohlkörper (2) hindurch einsehbar ist, **dadurch gekennzeichnet, dass** der Hohlkörper (2) ferner libellenförmig gestaltet ist und es sich bei dem Kennzeichnungselement (3) um einen Feststoff handelt und der Feststoff wenigstens ein Farbmittel aufweist.

2. Medizinisches Instrument (1) oder Teil eines medizinischen Instruments (1) nach Anspruch 1, **dadurch gekennzeichnet, dass** der Hohlkörper (2) mit dem darin enthaltenen Kennzeichnungselement (3) nach Art einer Röhrenlibelle, insbesondere Setzlibelle, oder Dosenlibelle, gestaltet ist.

3. Medizinisches Instrument (1) oder Teil eines medizinischen Instruments (1) nach Anspruch 1 oder 2, **dadurch gekennzeichnet, dass** der Hohlkörper (2) wenigstens abschnittsweise aus Glas oder Kunststoff besteht.

4. Medizinisches Instrument (1) oder Teil eines medizinischen Instruments (1) nach einem der vorhergehenden Ansprüche, **dadurch gekennzeichnet, dass** der Hohlkörper (2) wenigstens abschnittsweise in einem Hohlkörpergehäuse (4) eingebaut oder in eine Aufnahmeaussparung (6) eines Hohlkörpereinsatzes (7) eingefügt, insbesondere eingepresst, ist.

5. Medizinisches Instrument (1) oder Teil eines medizinischen Instruments (1) nach Anspruch 4, **dadurch gekennzeichnet, dass** das Hohlkörpergehäuse (4) wenigstens abschnittsweise lichtdurchlässig gestaltet ist, insbesondere wenigstens abschnittsweise aus lichtdurchlässigem Glas oder einem lichtdurchlässigen Kunststoff besteht, oder der Hohlkörpereinsatz (7) aus Kunststoff, einem Metall oder einer Metalllegierung besteht.

6. Medizinisches Instrument (1) oder Teil eines medizinischen Instruments (1) nach einem der vorhergehenden Ansprüche, insbesondere nach Anspruch 5, **dadurch gekennzeichnet, dass** der Hohlkörper (2), insbesondere zusammen mit dem Hohlkörpergehäuse (4) oder dem Hohlkörpereinsatz (7), in eine Aufnahmeaussparung (8) des medizinischen Instruments (1) oder des Teils eines medizinischen Instruments (1) eingefügt, insbesondere eingepresst, ist.

7. Medizinisches Instrument (1) oder Teil eines medizinischen Instruments (1) nach einem der vorhergehenden Ansprüche, **dadurch gekennzeichnet, dass** der wenigstens ein Farbmittel aufweisende Feststoff in Form von farbigen Partikeln, insbesondere in Form eines farbigen Pulvers oder farbigen Granulats, vorliegt.

8. Medizinisches Instrument (1) oder Teil eines medizinischen Instruments (1) nach einem der vorhergehenden Ansprüche 1 bis 6, **dadurch gekennzeichnet, dass** der wenigstens ein Farbmittel aufweisende Feststoff in Form eines farbigen, bemalten, lackierten oder bedruckten Glases, insbesondere in Form eines farbigen, bemalten, lackierten oder bedruckten Hinterglases, vorliegt.

9. Medizinisches Instrument (1) oder Teil eines medizinischen Instruments (1) nach einem der vorhergehenden Ansprüche, **dadurch gekennzeichnet, dass** es sich bei dem wenigstens einen Farbmittel um wenigstens einen Farbstoff, vorzugsweise ausgewählt aus der Gruppe bestehend aus Anthrachinonfarbstoffe, Azofarbstoffe, Dioxazinfarbstoffe, indigoide Farbstoffe, Metallkomplexfarbstoffe, Formazanfarbstoffe, Phthalocyaninfarbstoffe, Methinfarbstoffe, Direktfarbstoffe, Dispersionsfarbstoffe, Entwicklungs- oder Kupplungsfarbstoffe, kationische Farbstoffe, Küpenfarbstoffe, Beizenfarbstoffe, Lösungsmittelfarbstoffe, Reaktivfarbstoffe, Säurefarbstoffe, funktionelle Farbstoffe, Lebensmittelfarbstoffe, Vitalfarbstoffe, Anilinblau, Azokarmin wie Azokarmin G (Rosindulin), Bromcresolgrün, Coomassie-Brillant-Blau, Eisenhämatoxylin, Eosin, Fuchsin, Hämatoxylin, Kongorot, Lichtgrün, Methylenblau, Methylorange, Methylrot und Mischungen davon, handelt.

10. Medizinisches Instrument (1) oder Teil eines medizinischen Instruments (1) nach einem der Ansprüche 1 bis 8, **dadurch gekennzeichnet, dass** es sich bei dem wenigstens einen Farbmittel um ein Pigment, bevorzugt ausgewählt aus der Gruppe bestehend aus Titandioxid, Aluminium, Silber, Gold, Calciumcarbonat, Titandioxid, Eisenoxid, Pflanzenkohle und Mischungen davon, handelt.

11. Medizinisches Instrument (1) oder Teil eines medizinischen Instruments (1) nach einem der vorhergehenden Ansprüche, **dadurch gekennzeichnet, dass** der Feststoff mit Bildern, Logos, Zeichnungen, Schriftzügen oder anderen Zeichen oder Symbolen versehen ist.

12. Kit oder Set, aufweisend räumlich getrennt voneinander eine Vielzahl von medizinischen Instrumenten oder Teilen von medizinischen Instrumenten nach einem der vorhergehenden Ansprüche 1 bis 11, wobei sich die medizinischen Instrumente oder Teile von medizinischen Instrumenten in Bezug auf ihre Größe sowie in Bezug auf das Kennzeichnungselement voneinander unterscheiden.

## Claims

1. Medical instrument (1) or part of a medical instrument (1), wherein the medical instrument or the part of a medical instrument (1) has a closed hollow body (2) which is of at least partially light-permeable design, wherein the hollow body (2) contains a marking element (3), wherein the marking element (3) can be seen through the at least partially light-permeable hollow body (2), **characterized in that** the hollow body (2) is also designed in the form of a level and the marking element (3) is a solid and the solid comprises at least one colouring agent.

2. Medical instrument (1) or part of a medical instrument (1) according to Claim 1, **characterized in that** the hollow body (2) with the marking element (3) contained therein is designed in the manner of a tubular level, in particular striding level, or circular level.

3. Medical instrument (1) or part of a medical instrument (1) according to Claim 1 or 2, **characterized in that** the hollow body (2) at least partially consists of glass or plastic.

4. Medical instrument (1) or part of a medical instrument (1) according to one of the preceding claims, **characterized in that** the hollow body (2) is at least partially installed in a hollow body housing (4) or is inserted, in particular pressed, into a receiving cutout (6) in a hollow body insert (7) .

5. Medical instrument (1) or part of a medical instrument (1) according to Claim 4, **characterized in that** the hollow body housing (4) is of at least partially light-permeable design, in particular at least partially consists of light-permeable glass or a light-permeable plastic, or the hollow body insert (7) consists of plastic, a metal or a metal alloy.

6. Medical instrument (1) or part of a medical instrument (1) according to one of the preceding claims, in particular according to Claim 5, **characterized in that** the hollow body (2), in particular together with the hollow body housing (4) or the hollow body insert (7), is inserted, in particular pressed, into a receiving cutout (8) in the medical instrument (1) or in the part of a medical instrument (1).

7. Medical instrument (1) or part of a medical instrument (1) according to one of the preceding claims, **characterized in that** the solid comprising at least one colouring agent is in the form of coloured particles, in particular in the form of a coloured powder or coloured granular material.

8. Medical instrument (1) or part of a medical instrument (1) according to one of the preceding Claims 1 to 6, **characterized in that** the solid comprising at least one colouring agent is in the form of a coloured, painted, coated or printed glass, in particular in the form of a coloured, painted, coated or printed rear glass.

9. Medical instrument (1) or part of a medical instrument (1) according to one of the preceding claims, **characterized in that** the at least one colouring agent is at least one dye, preferably selected from the group consisting of anthraquinone dyes, azo dyes, dioxazine dyes, indigoid dyes, metal complex dyes, formazan dyes, phthalocyanine dyes, methine dyes, direct dyes, dispersion dyes, development or coupling dyes, cationic dyes, vat dyes, mordant dyes, solvent dyes, reactive dyes, acid dyes, functional dyes, food dyes, vital dyes, aniline blue, azocarmine such as azocarmine G (rosinduline), bromocresol green, Coomassie brilliant blue, iron haematoxylin, eosin, fuchsine, haematoxylin, Congo red, Light Green, methylene blue, methyl orange, methyl red and mixtures thereof.

10. Medical instrument (1) or part of the medical instrument (1) according to one of Claims 1 to 8, **characterized in that** the at least one colouring agent is a pigment, preferably selected from the group consisting of titanium dioxide, aluminium, silver, gold, calcium carbonate, titanium dioxide, iron oxide, vegetable black and mixtures thereof.

11. Medical instrument (1) part of a medical instrument (1) according to one of the preceding claims, **characterized in that** the solid is provided with images, logos, drawings, letterings or other signs or symbols.

12. Kit or set comprising, spatially separated from one another, a multiplicity of medical instruments or parts of medical instruments according to one of the preceding Claims 1 to 11, wherein the medical instruments or parts of medical instruments differ from one another in relation to their size and in relation to the marking element.

## Revendications

1. Instrument médical (1) ou partie d'un instrument médical (1), dans lequel l'instrument médical ou la partie d'un instrument médical (1) présente un corps creux fermé (2) qui est conçu au moins partiellement pour laisser passer la lumière, le corps creux (2) contenant un élément d'identification (3), l'élément d'identification (3) étant visible à travers le corps creux (2) conçu au moins partiellement pour laisser passer la lumière, **caractérisé en ce que** le corps creux (2) est en outre conçu en forme de nivelle et **en ce que** l'élément d'identification (3) est un solide et **en ce que** le solide comprend au moins un colorant.

2. Instrument médical (1) ou partie d'un instrument médical (1) selon la revendication 1, **caractérisé en ce que** le corps creux (2) avec l'élément d'identification (3) qu'il contient est conçu à la manière d'une nivelle tubulaire, en particulier d'une nivelle de pose ou d'une nivelle circulaire.

3. Instrument médical (1) ou partie d'un instrument médical (1) selon la revendication 1 ou la revendication 2, **caractérisé en ce que** le corps creux (2) est constitué au moins partiellement de verre ou de matière plastique.

4. Instrument médical (1) ou partie d'un instrument médical (1) selon l'une des revendications précédentes, **caractérisé en ce que** le corps creux (2) est monté au moins partiellement dans un boîtier de corps creux (4) ou est inséré, notamment par pression, dans un évidement de réception (6) d'un insert de corps creux (7).

5. Instrument médical (1) ou partie d'un instrument médical (1) selon la revendication 4, **caractérisé en ce que** le boîtier du corps creux (4) est conçu de manière à laisser passer la lumière au moins partiellement, en particulier est constitué au moins partiellement de verre laissant passer la lumière ou d'une matière plastique laissant passer la lumière, ou l'insert du corps creux (7) est constitué de matière plastique, d'un métal ou d'un alliage de métaux.

6. Instrument médical (1) ou partie d'instrument médical (1) selon l'une des revendications précédentes, en particulier selon la revendication 5, **caractérisé en ce que** le corps creux (2), en particulier avec le boîtier de corps creux (4) ou l'insert de corps creux (7), est inséré, en particulier pressé, dans un évidement de réception (8) de l'instrument médical (1) ou de la partie d'instrument médical (1).

7. Instrument médical (1) ou partie d'instrument médical (1) selon l'une des revendications précédentes, **caractérisé en ce que** le solide présentant au moins un colorant se présente sous forme de particules colorées, notamment sous forme de poudre colorée ou de granulés colorés.

8. Instrument médical (1) ou partie d'instrument médical (1) selon l'une des revendications précédentes 1 à 6, **caractérisé en ce que** le solide comportant au moins un colorant se présente sous la forme d'un verre coloré, peint, laqué ou imprimé, notamment sous la forme d'un verre arrière coloré, peint, laqué ou imprimé.

9. Instrument médical (1) ou partie d'instrument médical (1) selon l'une des revendications précédentes, **caractérisé en ce que** ledit au moins un colorant est au moins un colorant, de préférence choisi dans le groupe constitué par les colorants anthraquinoniques, les colorants azoïques, les colorants dioxaziniques, les colorants indigoïdes, les colorants à complexe métallique, les colorants formazan, les colorants phtalocyanine, les colorants méthiniques, les colorants directs, les colorants dispersés, les colorants de développement ou de liaison, les colorants cationiques, les colorants de cuve, les colorants de mordançage, les colorants de solvants, les colorants réactifs, les colorants acides, les colorants fonctionnels, les colorants alimentaires, les colorants vitaux, le bleu d'aniline, l'azocarmin tel que l'azocarmin G (insuline rose), le vert de bromocrésol, le bleu brillant de Coomassie, l'hématoxyline de fer, l'éosine, la fuchsine, l'hématoxyline, le rouge Congo, le vert de lumière, le bleu de méthylène, l'orange de méthyle, le rouge de méthyle, et les mélanges de ceux-ci.

10. Instrument médical (1) ou partie d'un instrument médical (1) selon l'une des revendications 1 à 8, **caractérisé en ce que** ledit au moins un colorant est un pigment, de préférence choisi dans le groupe constitué par le dioxyde de titane, l'aluminium, l'argent, l'or, le carbonate de calcium, le dioxyde de titane, l'oxyde de fer, le charbon végétal, et les mélanges de ceux-ci.

11. Instrument médical (1) ou partie d'un instrument médical (1) selon l'une des revendications précédentes, **caractérisé en ce que** le solide est pourvu d'images, de logos, de dessins, d'écritures ou d'autres signes ou symboles.

12. Kit ou ensemble comprenant, séparés les uns des autres dans l'espace, une pluralité d'instruments médicaux ou de parties d'instruments médicaux selon l'une des revendications 1 à 11 précédentes, lesdits instruments médicaux ou parties d'instruments médicaux étant différents les uns des autres en termes de taille ainsi qu'en termes d'élément d'identification.
